# EUROPEAN PATENT APPLICATION

(11) **EP 3 281 531 A1**
(43) Date of publication of application: **14.02.2018**
(21) Application number: 16776554.4
(22) Date of filing: 06.04.2016
(51) Int. Cl.: A23K 20/00, A23K 50/75

(54) **METHOD FOR IMPROVING FEED CONVERSION RATIO OF POULTRY AND METHOD FOR REARING POULTRY**

(30) Priority: 06.04.2015 US 201562143399 P
(71) Applicant: Asahi Calpis Wellness Co., Ltd., Shibuya-ku Tokyo 150-0022 (JP)
(72) Inventor: YASUDA Gentaro, Tokyo 150-0022 (JP); KATO Masaya, Peachtree City, Georgia 30269 (US)
(74) Representative: D Young & Co LLP
(86) International application number: PCT/JP2016/061240
(87) International publication number: WO 2016/163388

(57) **Abstract**

The invention of the application provides a method of breeding poultry or a method of improving a feed conversion ratio (FCR), characterized by comprising feeding *Bacillus coagulans* at a late stage of poultry production in breeding of the poultry, and further provides a feed additive composition for use in improving poultry FCR comprising *Bacillus coagulans* strain CP3425 (International Accession Number NITE BP-01693) or a derivative or variant thereof.

## Description

### TECHNICAL FIELD

The present invention relates to a method for improving a feed conversion ratio (FCR) of poultry and to a method for breeding poultry, both comprising feeding *Bacillus coagulans* to the poultry.

The present invention also relates to a feed additive composition to improve a FCR of poultry.

### BACKGROUND ART

Patent Literatures 1 to 4 are examples of the literature concerning improvement of the productivity of poultry, including improvement of a feed conversion ratio of poultry.

Patent Literature 1 describes a feed additive composition comprising a direct fed microbial in combination with a phytase derived from *Citrobacter spp.*

Patent Literature 2 describes a feed additive composition comprising a direct fed microbial mixed with a protease and a phytase.

Patent Literature 3 describes a feed additive composition comprising a direct fed microbial in combination with a protease, a xylanase, an amylase, and a phytase.

Patent Literatures 1 to 3 describe *Bacillus* bacteria, such as *Bacillus subtilis, Bacillus licheniformis, Bacillus amyloliquefaciens,* and *Bacillus cereus,* among direct-fed microbials.

Patent Literature 4 describes a method for breeding poultry for meat production comprising feeding a corn-soybean meal feed to the poultry, wherein the corn-soybean meal feed further contains *Bacillus licheniformis* PWD-1 keratinase in an amount effective for accelerating the body weight gain of the poultry for meat production.

Non-Patent Literature 1 describes effects of *Bacillus coagulans* ZJU0616 administered in different concentrations to Guangxi Yellow chickens in terms of, for example, growth performance, feed conversion ratio, and survival rate. When such microbial is fed at concentrations of 2.0 x 10⁶ cfu/g of feed and 5.0 x 10⁶ cfu/g of feed, the feed conversion ratio falls significantly and the survival rate becomes higher than control.

Non-Patent Literature 2 describes the growth performance of broilers and the effect of *Bacillus coagulans* on the intestinal flora. When the concentration of *B. coagulans* is 0.005% or 0.04%, respectively, FCR is significantly improved over a period of 21-42 days or over a period of 42 days.

### CITATION LIST

### Patent Literature

Patent Literature 1: JP 2014-509846 A
Patent Literature 2: JP 2014-508519 A
Patent Literature 3: JP 2014-507946 A
Patent Literature 4: JP 2006-506982 A

### Non-Patent Literature

Non-Patent Literature 1: X. Zhou et al., Poultry Science 2010, 89: 588-593
Non-Patent Literature 2: S.Y. Lin et al., Journal of Animal and Veterinary Advances 2011, 10 (1): 111-114

### SUMMARY OF INVENTION

An object of the present invention is to improve a feed conversion ratio of and a weight gain rate of poultry using a bacterium or bacteria of the genus *Bacillus,* when compared with prior art techniques.

The present inventors conducted intensive studies and, as a result, have now found that the significantly high feed conversion ratio and weight gain rate could be achieved by feeding one or two bacteria belonging to the genus *Bacillus* to poultry.

Accordingly, the invention includes characteristics as described below.
(1) A method for breeding poultry, characterized in that the method comprises feeding *Bacillus coagulans* to the poultry at a late stage of poultry production.
(2) A method for improving a feed conversion ratio (FCR) of poultry, characterized in that the method comprises feeding *Bacillus coagulans* to the poultry at a late stage of poultry production.
(3) The method of (1) or (2), wherein the *Bacillus coagulans* is fed in an amount of at least 5.0 x 10² cfu/g of feed.
(4) The method of any of (1) to (3), wherein the *Bacillus coagulans* is fed in combination with a finisher or withdrawal feed.
(5) The method of any of (1) to (4), wherein the poultry is for meat food.
(6) The method of any of (1) to (5), wherein the *Bacillus coagulans* is fed in combination with another probiotic.
(7) The method of (6), wherein the probiotic is *Bacillus subtilis.*
(8) The method of (7), wherein the *Bacillus subtilis* is *Bacillus subtilis* strain C-3102 (International Accession Number FERM BP-1096), or a derivative or variant thereof.
(9) The method of (7) or (8), wherein the *Bacillus subtilis* or the *Bacillus subtilis* strain C-3102 or derivative or variant thereof is continuously fed from before the late stage of poultry production.
(10) The method of (7) or (8), wherein the *Bacillus subtilis* or the *Bacillus subtilis* strain C-3102 or derivative or variant thereof is fed from the stage of neonatal poultry.
(11) The method of any of (1) to (10), wherein the *Bacillus coagulans* is *Bacillus coagulans* strain CP3425 (International Accession Number NITE BP-01693), or a derivative or variant thereof.
(12) The method of any of (1) to (11), wherein the *Bacillus coagulans* has an ability to improve FCR by at least 1-10% when compared with control that the *Bacillus coagulans* is not fed to.
(13) A feed additive composition for improving a feed conversion ratio (FCR) of poultry, comprising *Bacillus coagulans* strain CP3425 (International Accession Number NITE BP-01693), or a derivative or variant thereof.
(14) The feed additive composition of (13), further comprising *Bacillus subtilis.*
(15) The feed additive composition of (14), wherein the *Bacillus subtilis* is *Bacillus subtilis* strain C-3102 (International Accession Number FERM BP-1096), or a derivative or variant thereof.
(16) The feed additive composition of any of (13) to (15), which is used in the method according to any of (1) to (12).

### BRIEF DESCRIPTION OF DRAWINGS

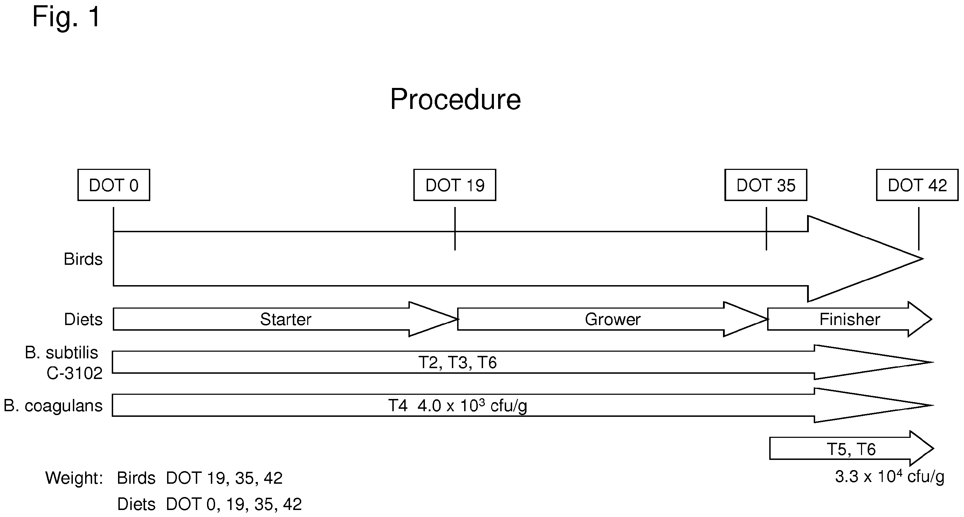

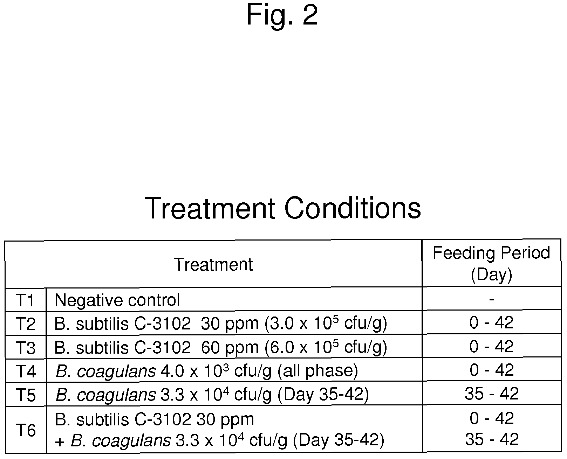

Fig. 1 depicts procedures for feeding samples to chickens (broilers) under different conditions during a period of from 0 day of age to 42 days of age as described in Examples below. DOT0, DOT19, DOT35, and DOT42 represent 0 day of age, 19 days of age, 35 days of age, and 42 days of age, respectively. Diets (feeds) indicate three types of feeds that are changed depending on the age of a bird in days; i.e., Starter; Grower, and Finisher, which are control samples consisting of a feed alone. T2, T3, T4, T5, and T6 indicate treatments shown in Fig. 2. Weight (weight measurement) indicates the weights of birds and the amounts of feeds consumed by birds measured at the indicated ages in days.
Fig. 2 shows treatment conditions (T1 to T6) to study effects of FCR improvement and weight gain in tests conducted in Examples below. T4, T5, and T6 indicate the conditions of the present invention.
Fig. 3 is a chart indicating day-by-day changes in weight gain of chickens tested in accordance with the procedures indicated in Fig. 1 using the treatment conditions T1 to T6 shown in Fig. 2.
Fig. 4 is a chart indicating day-by-day changes in FCR of chickens tested in accordance with the procedures indicated in Fig. 1 using the treatment conditions T1 to T6 shown in Fig. 2.
Fig. 5 shows the results of measurements of feed intake (kg), FCR, weight gain (kg), and variation of FCR in D35-42 at 19 days of age, 35 days of age, and 42 days of age, when tested in accordance with the procedures indicated in Fig. 1 using the treatment conditions. The data were statistically analyzed in the manner described below. The data were statistically evaluated by comparison of average values using the least significant difference test (Tukey's HSD test) and by general linear model using ANOVA.

### DESCRIPTION OF EMBODIMENTS

The present invention will be described in more detail.

### 1. Method for poultry breeding and method for FCR improvement

The invention provides a method for breeding poultry comprising feeding *Bacillus coagulans* to the poultry at a late stage of poultry production.

The invention also provides a method for improving a feed conversion ratio (FCR) of poultry comprising feeding *Bacillus coagulans* to the poultry at a late stage of poultry production.

In general, the term "feed conversion ratio (FCR)" used herein refers to a ratio of weight gain of animals to amount of consumed feed (i.e., weight gain (kg)/feed intake (kg)) in animals, or an amount of feed fed to animals needed to increase 1 kg of body weight. For example, the term "FCR2" implies that the amount of feed required is 2 kg. This suggests that, as the FCR value is smaller, the feed utilization becomes better. It is known that the FCR significantly varies depending on poultry types and breeding environments. In addition, because the FCR merely represents the amount of feed, if the FCR value is high, then the amount of reduced feed would become large even if the rate of FCR improvement is small. Normally, major economic effects can be expected if FCR is improved by 0.01, preferably 0.1 or more. When the rate of improvement is 1% or more, preferably 5% or more, and further preferably 7% or more, the effect of improvement is accepted as remarkable, although it depends on FCR values.

The term "poultry" used herein refers to fowl raised by a human(s) for commercial purposes. Preferable examples of poultry include fowl used for food, such as broilers, turkeys, geese, pheasants, and ducks (e.g., Peking duck).

The duration of poultry breeding is days of from 0 day after birth until the day of shipping. Such duration varies depending on poultry types and the time of shipping, and it is about 25 days to 170 days, although the duration is not limited thereto. Specifically, the duration of breeding is: for example, about 35 days to 60 days for broilers; about 140 days to 170 days for turkeys; about 40 days to about 50 days for Peking ducks; for about 25 days to about 30 days for geese; and about 120 days for pheasants.

A nutritious composition of a poultry feed (or a diet) is generally changed, for example, every 3 to 4 weeks during the poultry breeding period. In the case of broilers or turkeys among poultry, feeds fed from 0 day after birth are classified as a starter feed, a grower feed, and a finisher feed, and the nutritional compositions of such feeds, as well as the timing of feeding, vary. Adequate nutritional compositions of poultry feeds varied depending on the number of days after birth during the breeding period is described in: for example, Nutrient Requirements of Poultry, Animal Nutrient Handbook (2014, U.S.A.), National Research Council (NRC1994, U.S.A.), Section 12: Poultry Nutrition and Feeding, pp. 410-425; and E.A. Saleh et al., J. Appl. Poultry Res. 6: 290-297, 1997, and they can be employed in the invention.

Specifically, in case of broilers, for example, a starter feed is fed to the animals in 0 to 3 weeks of age (3200 kcal/kg energy and 22% protein), a grower feed is fed to the animals in 4 to 5 weeks of age (3200 kcal/kg energy and 20% protein), and a finisher feed is fed to the animals in 6 to 7 weeks of age (3200 kcal/kg energy and 18% protein).

In case of turkeys, for example, a starter feed is fed to the male and female animals in 0 to 4 weeks of age (2800 kcal/kg energy and 28% protein), a grower feed is fed to the male and female animals in 4 to 8 weeks of age (2900 kcal/kg energy and 26% protein), another grower feed is fed to the male animals in 8 to 12 weeks of age and to the female animals in 8 to 11 weeks of age (3000 kcal/kg energy and 22% protein), yet another grower feed is fed to the male animals in 12 to 16 weeks of age and to the female animals in 11 to 14 weeks of age (3100 kcal/kg energy and 19% protein), a finisher feed is fed to the male animals in 16 to 20 weeks of age and to the female animals in 14 to 17 weeks of age (3200 kcal/kg energy and 16.5% protein), and another finisher feed is fed to the male animals in 20 to 24 weeks of age and to the female animals in 17 to 20 weeks of age (3300 kcal/kg energy and 14% protein).

The examples of broiler and turkey feeds are based on NRC1994 (U.S.A., *ibid*)*.*

Examples of nutrition as protein/amino acid sources include: plant sources, such as soybean meal, cotton seed meal, phosphate seed meal, alfalfa meals, and corn gluten meal; and animal sources, including meat products, such as fish meal and animal byproducts. An example of a carbonate source is yellow corn. Other examples include fats and oils, minerals, and vitamins.

Commercially available poultry feeds may be used during poultry breeding.

Poultry is bred in a closed space within a given area (i.e., a pen) at an automatically regulated temperature while feeding the feeds with different nutritional compositions daily depending on the breeding stages. It is necessary for the poultry to be capable of freely drinking water during breeding.

Broilers are used as examples of poultry in the Examples below. During breeding, a starter feed was fed to the animals in 0 to 19 days of age, a grower feed was fed to the animals in 19 to 35 days of age, and a finisher feed was fed to the animals in about 35 to 42 days of age.

The methods of the invention (i.e., the method for breeding and the method for improving a feed conversion ratio) comprise feeding *Bacillus coagulans* to poultry at a late stage of poultry production.

It is known that, during "the late stage of production," the feed conversion ratio is lowered even if a broiler is sufficiently fed, and such phenomenon is generally observed in poultry with higher days of age (which may optionally be expressed by "weeks of age"). As used herein, the period of from the stage when broiler experiences such phenomenon until the stage when broiler is shipped is referred to as "the late stage of production." One example of the late stage of production may be a period during which a finisher feed is fed. In the case of broiler breeding, for example, the late stage of production is a period of from approximately 35 days of age until shipping (e.g., 42 days of age) (see Fig. 1) or a period of from approximately 42 days of age until shipping (e.g., 56 days of age). In the case of production of other poultry for food, alternatively, the period of from approximately 30 to 60 days before shipping until the day of shipping may be the late stage of production. In the case of turkeys, the late stage of production is, for example, a period of from 16 weeks of age to 24 weeks of age (112 days of age to 168 days of age). In the case of pheasants, the late stage of production is, for example, a period of from 9 weeks of age to 17 weeks of age (63 days of age to 119 days of age).

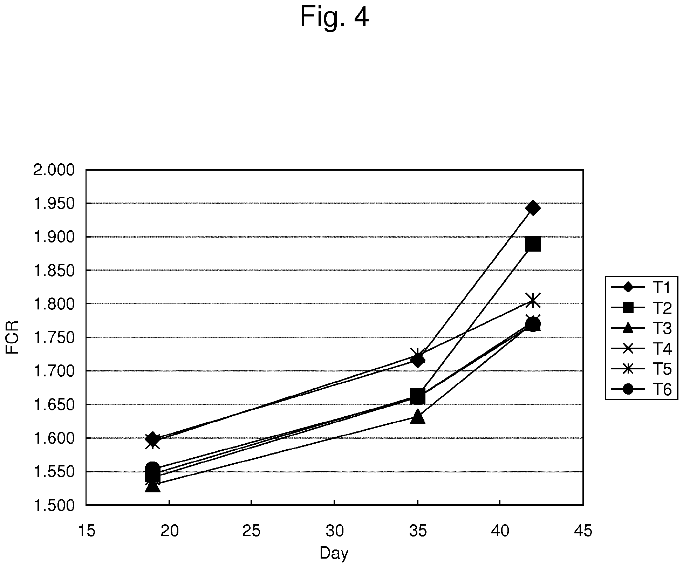

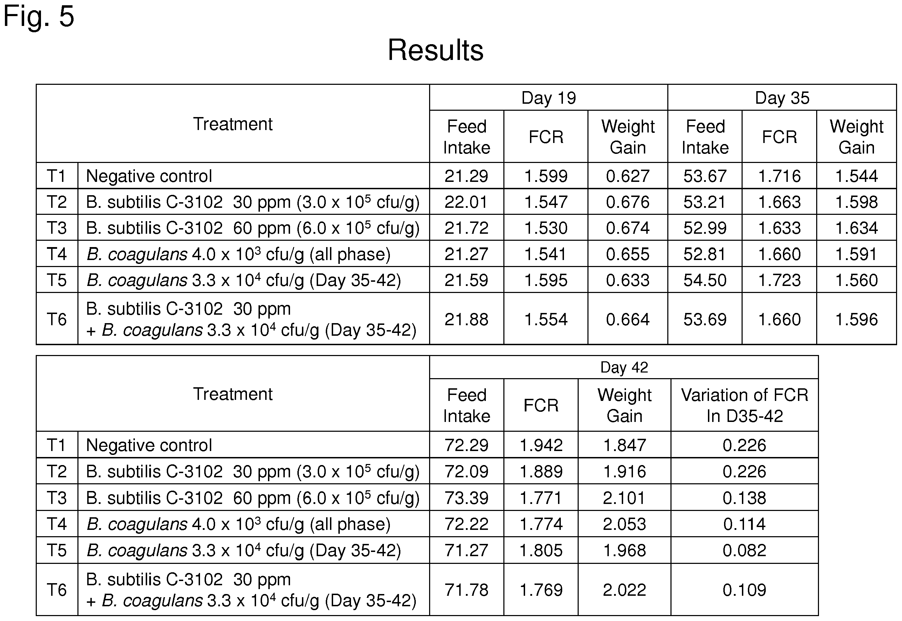

According to the method of the invention, sufficient effects can be attained when *Bacillus coagulans* is fed at least at a late stage of production (see, for example, T5 and T6 in Fig. 4 and Fig. 5). Also, *Bacillus coagulans* may be fed from before the late stage of production or from the neonatal chick stage (see T4 in Fig. 4 and Fig. 5).

According to the invention, excellent feed utilization can be achieved by feeding *Bacillus coagulans* at a low concentration, such as 1.0 x 10³ cfu/g of feed to 4.0 x 10³ cfu/g of feed, and even only at the late stage of production (see "8. Results" in the Examples below).

*Bacillus coagulans* is mixed with a general poultry feed or a feed that is generally known to be fed at a late stage of production, such as a finisher feed or withdrawal feed, and the mixture is then fed to poultry.

Ingredients of poultry feeds are not particularly limited, provided that the ingredients are commonly used. Examples of the ingredients include: protein sources, such as soybean meal, fish meal, meat meal, wheat meal, rapeseed, and canola; carbohydrate sources, such as corn, oat, barley, sorghum, wheat, triticale, rye, and rice; and additives, such as vitamins, minerals, and oils and fats.

The amount of *Bacillus coagulans* to be added to feeds is at least approximately 5.0 x 10² cfu/g of feed, for example, at least approximately (1.0 to 4.0) x 10³ cfu/g of feed, preferably at least approximately 3.0 ×x 10³ cfu/g of feed or approximately 4.0 x 10³ cfu/g of feed. While the upper limit is not particularly limited, for example, the amount can be approximately 1.0 x 10⁷ cfu/g of feed to approximately 1.0 x 10⁹ cfu/g of feed.

*Bacillus coagulans* may be cultured using conditions as described in literature. An example of culture methods is as described below.

*Bacillus coagulans* may be cultured in a liquid medium (pH 7.2 to 7.4) containing carbon sources, nitrogen sources, inorganic matter, and the like under aerobic conditions at 37°C to 38°C for at least about 12 hours to about 3 days with mild agitation, and the resultant culture may be centrifuged to collect the microbial cells (see, for example, H. K. Wang et al., Food Technol. Biotechnol. 51 (1): 78-83, 2013). Examples of carbon sources include assimilable carbon sources, such as glucose, fructose, sucrose, starch, and molasses. Examples of nitrogen sources include peptone, meat extract, yeast extract, casein hydrolysate, and ammonium sulfate. In addition, where needed, salts as inorganic matter, such as phosphate, magnesium, potassium, sodium, calcium, iron, and manganese, vitamins, amino acids, an antifoaming agent, a surfactant, or the like may be added .

The collected microbial cells may be prepared in any form, such as culture, concentrate, dry matter, liquid, grain, powder, pellet, bar, or sphere.

Any strain of the *Bacillus coagulans* may be used, provided that such strain yields an effect of the invention, that is, an effect of improving the poultry feed conversion ratio (FCR) when the strain is fed to poultry at a late stage of production. Examples of the *Bacillus coagulans* strain include, but are not limited to, *Bacillus coagulans* strain CP3425, orderivatives or variants thereof.

The *Bacillus coagulans* strain CP3425 was deposited at the Patent Microorganisms Depositary, the National Institute of Technology and Evaluation (Room 122, 2-5-8 Kazusakamatari, Kisarazu-shi, Chiba, 292-0818, Japan), which is an international depositary authority under the terms of the Budapest Treaty, on August 13, 2013, under International Accession Number NITE BP-01693.

The derivatives or variants may be obtained by methods comprising: for example, culturing a parent strain in the presence of a chemical mutagen, such as nitrosoguanidine, nitrosourea, methyl ethanesulfonate, or a derivative thereof; or irradiating a cultured parent strain with a high energy ray, such as UV or X-ray. A strain having the effect of improving the poultry feed conversion ratio (FCR) when fed to poultry at a late stage of production is selected from the obtained strains.

According to another embodiment, the invention comprises feeding the above-mentioned *Bacillus coagulans* in combination with another probiotics.

Preferably, examples of such probiotic include bacteria of the genus *Lactobacillus* and bacteria of the genus *Bacillus,* more preferably bacteria of the genus *Bacillus.*

Specific examples of bacteria of the genus *Bacillus* include *Bacillus subtilis, Bacillus licheniformis, Bacillus amyloliquefaciens,* and *Bacillus cereus,* preferably *Bacillus subtilis.*

Examples of bacteria of the genus *Lactobacillus* include *Lactobacillus bulgaricus, Lactobacillus casei, Lactobacillus acidophilus, Lactobacillus plantarum, Lactobacillus delbrueckii, Lactobacillus buchneri, Lactobacillus helveticus,* and *Lactobacillus fermentum.*

In culture of bacteria of the genus *Bacillus* and of the genus *Lactobacillus,* a liquid or solid medium containing carbon sources, nitrogen sources, inorganic matter, and the like may be used. Examples of carbon sources include assimilable carbon sources, such as glucose, fructose, sucrose, starch, and molasses. Examples of nitrogen sources include peptone, meat extract, casein hydrolysate, and ammonium sulfate. In addition, where needed, salts as inorganic matter, such as phosphate, magnesium, potassium, sodium, calcium, iron, and manganese, vitamins, amino acids, an antifoaming agent, a surfactant, or another substance may be added. Culture is preferably conducted under aerobic conditions. The initial pH of a medium is 5 to 9, preferably 6 to 8, culture temperature is 20°C to 50°C, preferably 35°C to 40°C, and the culture period is, for example, 12 hours to 7 days. After the completion of culture, microbial cells can be collected by a separation method, such as centrifugation. The collected cells may be prepared in any form, such as culture, concentrate, dry matter, liquid, grain, powder, pellet, bar, or sphere.

A preferable example of *Bacillus subtilis* is *Bacillus subtilis* strain C-3102, or a derivative or variant thereof.

The *Bacillus subtilis* strain C-3102 (International Accession Number FERM BP-1096) was originally deposited on December 25, 1985 under F.R.I. Deposit No. 8584 at the International Patent Organism Depositary, National Institute of Advanced Industrial Science and Technology (the old name when deposited: Fermentation Research Institute, Agency of Industrial Science and Technology, Ministry of International Trade and Industry) located at 1-1-1 Higashi, Tsukuba, Ibaraki, Japan (the old address when deposited: 1-1-3 Higashi, Yatabe, Tsukuba, Ibaraki, Japan), and the strain C-3102 was then transferred to the international deposit on June 28, 1986 at the same depositary under Accession No. FERM BP-1096 (the old deposit number when transferred: F.R.I. Deposit No. 1096). This strain is also described in, for example, JP S63-209580 A (1988) and JP S62-232343 A (1987).

The above-mentioned derivatives or variants may be obtained in a similar manner to the method for preparation described above.

According to another embodiment, the invention comprises continuously feeding the probiotic, the bacterium of the genus *Bacillus,* the *Bacillus subtilis,* or the *Bacillus subtilis* strain C-3102 from before the late stage of poultry production, or alternatively, feeding the probiotic, the bacterium of the genus *Bacillus,* the *Bacillus subtilis,* or the *Bacillus subtilis* strain C-3102 from the stage of a neonatal chick. When the *Bacillus subtilis* strain C-3102 is to be fed, a commercially available product, such as Calsporin™ (Asahi Calpis Wellness Co. Ltd., Tokyo, Japan), may be used.

The amount of the probiotic, the bacterium of the genus *Bacillus,* the *Bacillus subtilis,* or the *Bacillus subtilis* strain C-3102 to be added to the feed is at least approximately 3.0 x 10⁵ cfu/g of feed, such as at least approximately (3.0 to 10.0) x 10⁵ cfu/g of feed, preferably at least approximately 5.0 x 10⁵ cfu/g of feed. While the upper limit is not particularly limited, for example, the amount may be from approximately 1.0 x 10⁷ cfu/g of feed to approximately 1.0 x 10⁹ cfu/g of feed.

According to a preferable embodiment of the invention, the *Bacillus coagulans* strain CP3425 (International Accession Number NITE BP-01693) or derivative or variant thereof, as the *Bacillus coagulans,* is fed to poultry in combination with the *Bacillus subtilis* strain C-3102 or derivative or variant thereof.

The results shown in Fig. 5 indicates that *Bacillus coagulans* used in the invention is capable of improving FCR by at least 1% to 10%, compared with a control group to which no *Bacillus coagulans* is fed.

### 2. Feed additive composition

The invention further provides a feed additive composition to improve the poultry feed conversion ratio (FCR) that comprises the *Bacillus coagulans* strain CP3425 (International Accession Number NITE BP-01693) or a derivative or variant thereof.

According to one embodiment of the invention, preferably the feed additive composition further comprises *Bacillus subtilis,* wherein the *Bacillus subtilis* itself has an effect of improving poultry FCR.

Preferably, the *Bacillus subtilis* is *Bacillus subtilis* strain C-3102 (International Accession Number FERM BP-1096) or a derivative or variant thereof.

According to another embodiment of the invention, the feed additive composition may be used in the method of the invention described in section 1 above.

The feed additive composition of the invention may be either in a solid form or in a liquid form. Examples of the solid form include powders, granules, and pellets. An example of the liquid form is a suspension. The composition may be formulated in such form by optionally containing a carrier, an excipient, an adjuvant, or the like that contains a substance approved for poultry.

The feed additive composition comprises, as an active ingredient, the *Bacillus coagulans* strain CP3425 (International Accession Number NITE BP-01693) or a derivative or variant thereof. The content is, for example, at least approximately 1.0 x 10⁴ cfu/g of the composition to approximately 1.0 x 10¹² cfu/g of the composition, although the content is not limited thereto.

In addition, the content of *Bacillus subtilis* in the feed additive composition is, for example, at least approximately 1.0 x 10³ cfu/g of the composition to approximately 1.0 x 10¹² cfu/g of the composition, although the content is not limited thereto.

The feed additive composition of the invention is added to the feed as described in section 1 above and fed to poultry. A preferable amount thereof added is 0.001% by weight to 1% by weight relative to the feed, although the amount is not limited thereto.

### EXAMPLES

The present invention will be more specifically described with reference to the following examples; however, the scope of the invention is not limited to the examples.

### [Test for comparison of performance of broilers fed with Bacillus coagulans CP3425]

### 1. Objective of experiment

The objective of the experiment was to evaluate the effects of *Bacillus coagulans* strain CP3425 on feed conversion and weight gain of male broilers raised to 42 days of age.

### 2. Description of treatment

Animals used in experiments were 20 male broilers per experimental unit in the 48 experimental units (i.e., "pens"). Treatments were replicated in 8 blocks, and 6 treatments (Fig. 2) were randomized within each block. A randomization procedure for pen assignment for treatments and blocks was done by Southern Poultry Research, Inc. (U.S.A.). The pen diagrams and treatment assignments were included in the source data. All pens were serially numbered and identified on pen cards.

### 3. Materials and methods

Time line:
   Date of initiation (Start): December 11, 2014
   Date of completion (Finish): January 22, 2015
Animals:
   A total of 960, one-day-old Cobb X Cobb male broiler chicks
   20 birds/pen, 8 pens /treatment, 0.83 ft²/bird
Litter:
   New wood shavings as bedding with thickness of approximately 4 inches
Feeds (or Diets):
   No anti-coccidial drug or AGP was added to the feeds.
Test article:
   *Bacillus subtilis* C-3102
   *Bacillus coagulans* CP3425

### 4. Experimentation procedure

The procedure is shown in Fig. 1.

### Treatment (T):

T1: Negative control
T2: strain: *Bacillus subtilis* C-3102 (3.0 x 10⁵ cfu/g of feed), feeding period: Day 0 to Day 42
T3: strain: *Bacillus subtilis* C-3102 (6.0 x 10⁵ cfu/g of feed), feeding period: Day 0 to Day 42
T4: strain: *Bacillus coagulans* CP3425 (4.0 x 10³ cfu/g of feed), feeding period: Day 0 to Day 42
T5: strain: *Bacillus coagulans* CP3425 (3.3 x 10⁴ cfu/g of feed), feeding period: Day 35 to Day 42
T6: strains: *Bacillus subtilis* C-3102 (3.0 x 10⁵ cfu/g of feed), feeding period: Day 0 to Day 42; and *Bacillus coagulans* CP3425 (3.3 x 10⁴ cfu/g of feed), feeding period: Day 35 to Day 42
T1 to T6 are also indicated in Fig. 2.

### 5. Description and management of floor pen

The floor pen house for chickens is a temperature-controlled building, which has concrete floors and solid sidewalls.

The chicken house used for the experiment was divided into pens of equal size and the pens were arranged along a central island. The chickens were kept in 48 floor pens. The area of each floor pen was 4.5 ft × 4.5 ft = 20 ft² (1.86 m²), and wood shavings were bedded with thickness of approximately 4 inches. The stocking density, after subtracting out for equipment, was 0.83 ft²/bird. Each pen had 2.5-feet-high side walls covered with one poultry netting to prevent bird migration.

The temperature of the building was monitored. Environmental conditions during the trial were appropriate to the age of the animals. Fluorescent lighting was provided on top of the pens, so as to obtain light. Lighting was provided for 24 hours every day from Day 1 to Day 42.

Animals were allowed to freely (*ad libitum*) intake feeds in one tube-type feeds container per pen. Also, one nipple drinker line was provided for each pen so as to allow the animals to freely (*ad libitum*) drink water.

Throughout the experiments, the standard floor pen management practices were used. The animals and the housing facilities were inspected by the manager of the trial site and the workers of the broiler house twice a day, Observing and recording the general health conditions, constant supply of feeds and water, and temperature. All dead chickens were removed and unexpected events were recognized. The chickens that died during the experiments were recorded on the Daily Mortality Record, and were not replaced. Pen number, date of mortality, sex, body weight, and the results of diagnosis were recorded.

### 6. Chickens

Broilers (Day of hatch male Cobb 500) were obtained from the Cobb-Vantress hatchery, Cleveland, GA (U.S.A.). A total of 960 chickens were accommodated. Information on all test animals and extra chickens was recorded on the animal disposition form. The broilers were not vaccinated with a coccidiosis vaccine before they were accommodated. 12 chickens were accommodated in each pen. The weights of chickens in the pens were recorded on Day 0, Day 19, Day 35, and Day 42.

### 7. Feeds

All the feeds were the products of SPR CENTRE (UK). No anti-coccidial drugs or AGP were added to the feeds. All feeds were fed in the form of crumbles/pellets.

All feeds in the pens were weighed. Starter feed was fed from Day 0 to Day 19, and the non-consumed starter feed was weighed and discarded on Day 19. Then, grower feed was fed up to Day 35, and the non-consumed grower feed was weighed and discarded on Day 35. Subsequently, finisher feed was fed up to Day 42, and the non-consumed finisher feed was weighed and discarded on Day 42.

### 8. Results

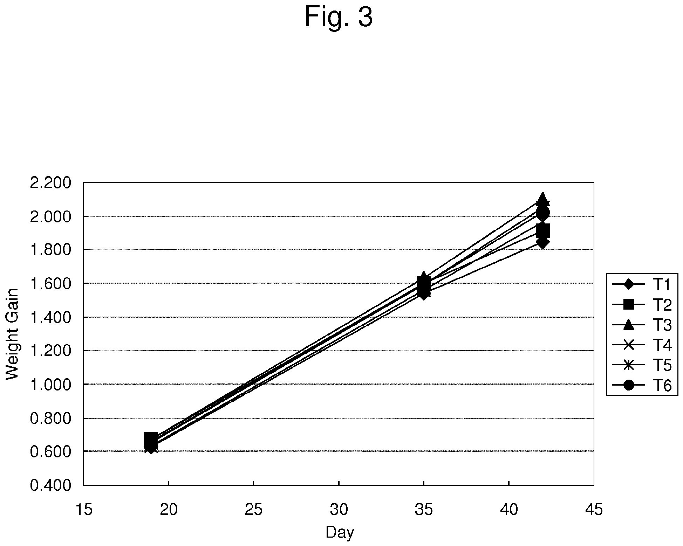

The results are shown in Figs. 3, 4, and 5.

The results are summarized as follows.

(1) FCR was improved by feeding *Bacillus coagulans* strain CP3425 throughout all stages (i.e., the whole period).

(2) FCR was improved by feeding *Bacillus coagulans* strain CP3425 during the period of from Day 35 to Day 42 (D35-42; i.e., the late stage of production).

(3) FCR of D35-42 (i.e., the late stage of production) was improved by feeding *Bacillus coagulans* strain CP3425 in combination with another *Bacillus* probiotic *(Bacillus subtilis* strain C-3102).

From the results of the experiments described above, it is found that the change in FCR of between D35-42 (i.e., the late stage of production) shown in Fig. 5 represents a difference between FCR of Day 35 and FCR of Day 42, and a method of treatment that gives a lesser degree of changes in FCR during the above-mentioned period (which is the late stage of production), i.e., in which an increase in FCR is suppressed, contributes to improvement of poultry meat production. Among the 6 treatment methods (T1 to T6) described above, T5 yielded the smallest change, and the extent of changes successively increased in the order of T6, T4, T3, and T1=T2. It has now been found that particularly T5 and T6, i.e. that are methods of treatment in which *B. coagulans* is fed only at t the late stage of production, enhance the suppression of an increase in FCR, thereby contributing to improvement of meat production, wherein T5 is a method of treatment in which *B. coagulans* is fed only at the late stage of production in breeding of poultry, and wherein T6 is a method of treatment in which *B. coagulans* (fed only at the late stage of production) is fed in combination with *B. subtilis* (fed throughout all stages of production).

In addition, the use of the *B. coagulans* strain CP3425 and the use of the *B. coagulans* strain CP3425 in combination with the *B. subtilis* strain C-3102 in the experiments above were found to have effects of significantly improving FCR at the time of poultry breeding, as demonstrated by the results attained by T4, T5, and T6.

### INDUSTRIAL APPLICABILITY

The industrial utility of the present invention is so great since FCR is significantly improved when breeding poultry for meat production, in comparison with conventional techniques.

### REFERENCE TO DEPOSITED BIOLOGICAL MATERIAL

The *Bacillus subtilis* strain C-3102 was domestically deposited on December 25, 1985 under F.R.I. Deposit No. 8584 at the International Patent Organism Depositary, National Institute of Advanced Industrial Science and Technology located at Tsukuba Central 6, 1-1-1 Higashi, Tsukuba, Ibaraki, 305-8566, Japan (the old address when deposited: 1-1-3 Higashi, Yatabe, Tsukuba, Ibaraki, 305, Japan), and the strain C-3102 was then transferred to the international deposit under the terms of the Budapest Treaty on June 28, 1986 at the same depositary under Accession No. FERM BP-1096 (the old deposit number when transferred: F.R.I. Deposit No. 1096). At present, this deposited strain is stored at the International Patent Organism Depositary, National Institute of Technology and Evaluation (NITE-IPOD) (Room 120, 2-5-8 Kazusakamatari, Kisarazu-shi, Chiba, 292-0818, Japan).

The *Bacillus coagulans* strain CP3425 was deposited on August 13, 2013 at the International Patent Microorganisms Depositary, National Institute of Technology and Evaluation (*ibid*)*,* which is the international depositary authority under the terms of the Budapest Treaty, under International Accession Number: NITE BP-01693.

## Claims

1. A method for breeding poultry, **characterized in that** the method comprises feeding *Bacillus coagulans* to the poultry at a late stage of poultry production.

2. A method for improving a feed conversion ratio (FCR) of poultry, **characterized in that** the method comprises feeding *Bacillus coagulans* to the poultry at a late stage of poultry production.

3. The method of claim 1 or 2, wherein the *Bacillus coagulans* is fed in an amount of at least 5.0 x 10² cfu/g of feed.

4. The method of any one of claims 1-3, wherein the *Bacillus coagulans* is fed in combination with a finisher or withdrawal feed.

5. The method of any one of claims 1-4, wherein the poultry is for meat food.

6. The method of any one of claims 1-5, wherein the *Bacillus coagulans* is fed in combination with another probiotic.

7. The method of claim 6, wherein the probiotic is *Bacillus subtilis.*

8. The method of claim 7, wherein the *Bacillus subtilis* is *Bacillus subtilis* strain C-3102 (International Accession Number FERM BP-1096), or a derivative or variant thereof.

9. The method of claim 7 or 8, wherein the *Bacillus subtilis* or the *Bacillus subtilis* strain C-3102 or derivative or variant thereof is continuously fed from before the late stage of poultry production.

10. The method of claim 7 or 8, wherein the *Bacillus subtilis* or the *Bacillus subtilis* strain C-3102 or derivative or variant thereof is fed from the stage of neonatal poultry.

11. The method of any one of claims 1-10, wherein the *Bacillus coagulans* is *Bacillus coagulans* strain CP3425 (International Accession Number NITE BP-01693), or a derivative or variant thereof.

12. The method of any one of claims 1-11, wherein the *Bacillus coagulans* has an ability to improve FCR by at least 1-10% when compared with control that the *Bacillus coagulans* is not fed to.

13. A feed additive composition for improving a feed conversion ratio (FCR) of poultry, comprising *Bacillus coagulans* strain CP3425 (International Accession Number NITE BP-01693), or a derivative or variant thereof.

14. The feed additive composition of claim 13, further comprising *Bacillus subtilis.*

15. The feed additive composition of claim 14, wherein the *Bacillus subtilis* is *Bacillus subtilis* strain C-3102 (International Accession Number FERM BP-1096), or a derivative or variant thereof.

16. The feed additive composition of any one of claims 13-15, which is for use in the method according to any one of claims 1-12.
